(19) Europäisches Patentamt
European Patent Office
Office européen des brevets

(11) **EP 3 885 765 B1**

(12) **FASCICULE DE BREVET EUROPEEN**

(45) Date de publication et mention
de la délivrance du brevet:
**01.05.2024   Bulletin 2024/18**

(21) Numéro de dépôt: **21163844.0**

(22) Date de dépôt: **21.03.2021**

(51) Classification Internationale des Brevets (IPC):
**G01N 33/49** *(2006.01)*      **G01N 21/17** *(2006.01)*
**G01N 29/24** *(2006.01)*      **A61B 5/00** *(2006.01)*
**G01N 29/02** *(2006.01)*      **A61B 5/145** *(2006.01)*

(52) Classification Coopérative des Brevets (CPC):
**G01N 21/1702; A61B 5/0095; A61B 5/14532;**
**G01N 21/1717; G01N 29/02; G01N 29/2418;**
**G01N 29/2425;** G01N 33/49; G01N 2021/1731;
G01N 2291/02466

(54) **DISPOSITIF DE DÉTECTION D'UN ANALYTE PAR DÉTECTION PHOTOACOUSTIQUE**

VORRICHTUNG ZUM NACHWEIS EINES ANALYTEN MITTELS PHOTOAKUSTISCHER DETEKTION

ANALYTE DETECTION DEVICE USING PHOTOACOUSTIC DETECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **24.03.2020   FR 2002883**

(43) Date de publication de la demande:
**29.09.2021   Bulletin 2021/39**

(73) Titulaire: **Commissariat à l'Energie Atomique et aux Energies Alternatives
75015 Paris (FR)**

(72) Inventeurs:
• **COUTARD, Jean-Guillaume**
  **38054 GRENOBLE CEDEX 09 (FR)**
• **DURAFFOURG, Laurent**
  **38054 GRENOBLE CEDEX 09 (FR)**
• **GLIERE, Alain**
  **38054 GRENOBLE CEDEX 09 (FR)**
• **TEULLE, Alexandre**
  **38054 GRENOBLE cedex 09 (FR)**

(74) Mandataire: **INNOV-GROUP
310, avenue Berthelot
69372 Lyon Cedex 08 (FR)**

(56) Documents cités:
**US-A1- 2008 011 055     US-A1- 2019 159 705**

## Description

## DOMAINE TECHNIQUE

Le domaine technique de l'invention est la détection d'un analyte selon le principe de la détection photoacoustique.

## ART ANTERIEUR

La détection photoacoustique est basée sur la détection d'une onde acoustique générée sous l'effet de l'absorption, par un milieu analysé, d'une onde incidente électromagnétique impulsionnelle ou modulée en amplitude. L'onde acoustique est formée suite à un échauffement de molécules absorbantes, présentes dans le milieu analysé, sous l'effet de l'absorption de l'onde incidente. L'échauffement entraîne une dilatation thermique modulée du milieu, cette dernière étant à l'origine de l'onde acoustique.

La détection photoacoustique peut être spécifique à un analyte particulier, en ajustant la longueur d'onde de l'onde incidente électromagnétique à une longueur d'onde d'absorption de l'analyte. La détection photoacoustique a été ainsi été appliquée pour détecter des espèces gazeuses dans un gaz, ou pour détecter la présence de molécules particulières dans des tissus biologiques. Le document US2008011055 A1 décrit un dispositif de détection photoacoustique appliqué à un liquide. La longueur d'onde de l'onde incidente se situe fréquemment dans le l'infra-rouge.

La détection photoacoustique constitue alors une technique d'analyse non invasive, pouvant être mise en oeuvre dans des milieux diffusants ou opaques.

Des applications de détection photoacoustique à des tissus biologiques sont par exemple décrits dans les publications suivantes :

- Bauer AJ. "IR-spectroscopy for skin in vivo : Optimal skin sites and properties for non-invasive glucose measurement by photoacoustic and photothermal spectroscopy" ; Journal of biopohtonics 11 (2018) ;
- "Windowless ultrasound photoacoustic cell for in-vivo mid-IR spectroscopy of human epidermis : Low interférence by changes of air pressure, température, and humidity caused by skin contact opens the possibility for a non-invasive monitoring of glucose in the interstitial fluid", Rev. Sci. Instrum. 84, 084901 (2013); et
- US2019159705 A1 qui divulgue le préambule de la revendication 1.

Dans ces publications, on utilise une source de lumière laser impulsionnelle, activée selon une fréquence comprises entre plusieurs dizaines de Hz et de plusieurs dizaines de kHz. L'objectif est d'estimer une concentration de glucose dans le fluide interstitiel corporel, à une profondeur comprise entre 10 $\mu$m et 50 $\mu$m sous la peau d'un utilisateur. On utilise pour cela un dispositif de détection photoacoustique placé contre la peau d'un utilisateur.

Les inventeurs ont cherché à améliorer les dispositifs existants, de façon à adresser des profondeurs plus importantes, et augmenter l'amplitude de l'onde acoustique détectée.

## EXPOSE DE L'INVENTION

Un premier objet de l'invention est un dispositif de détection photoacoustique, destiné à être appliqué, par une face de contact, contre un milieu à analyser, le dispositif comportant :

- une cavité creuse, débouchant sur une première ouverture, la première ouverture étant ménagée dans la face de contact;
- une source de lumière, impulsionnelle ou modulée en amplitude, configurée pour émettre une onde lumineuse incidente, dans une bande spectrale d'émission, à travers la cavité, jusqu'à la première ouverture ;
- un transducteur acoustique relié à la cavité, et configuré pour détecter une onde photoacoustique s'étendant à travers la cavité;

de telle sorte que sous l'effet d'une illumination du milieu par l'onde lumineuse incidente, le transducteur acoustique détecte une onde acoustique produite par un échauffement du milieu. Le dispositif peut comporter l'une quelconque des caractéristiques suivantes, prises isolément ou selon les combinaisons techniquement réalisables.

- la source de lumière est une source laser ;
- le volume de la cavité est inférieur à 50 $\mu$L (microlitre) ou à 30 $\mu$L. Il peut être compris entre 5 $\mu$L et 30 $\mu$L.
- le dispositif comporte un tube ouvert, s'étendant à partir de la cavité et débouchant sur de l'air s'étendant à l'extérieur de la cavité, selon une longueur comprise entre 1 et 20 mm, le diamètre du tube étant tel que :

  • lorsque le volume de la cavité est inférieur ou égal à 15 $\mu$L, le diamètre du tube est compris entre 150 $\mu$m et 300 $\mu$m.
  • lorsque le volume de la cavité est compris entre 15 $\mu$L et 30 $\mu$L, le diamètre du tube est compris entre 200 $\mu$m et 350 $\mu$m.
  • lorsque le volume de la cavité est supérieur à 30 $\mu$L, le diamètre du tube est compris entre 250 $\mu$m et 500 $\mu$m.

- la cavité est délimitée par une paroi transversale et une paroi latérale, la paroi latérale s'étendant entre la paroi transversale et la face de contact ;
- le tube s'étend à travers à partir la paroi transversale ou à travers la paroi latérale ;

3      **EP 3 885 765 B1**      4

- le dispositif comporte un canal acoustique s'étendant entre le transducteur acoustique et la cavité ;
- le dispositif comporte un composant optique configuré pour diriger l'onde lumineuse incidente, émise par la source de lumière, vers la première ouverture, le composant optique pouvant être un réflecteur ;
- la source de lumière est une source laser, le dispositif pouvant alors comporter un support s'étendant de la face de contact jusqu'à la source de lumière, le support étant configuré pour conduire une chaleur émise par le milieu analysé, vers la source de lumière ;
- le dispositif comporte un support s'étendant de la face de contact jusqu'à la source de lumière, le support étant configuré pour conduire une chaleur émise par le milieu analysé, vers la source de lumière. Le support comporte un métal thermiquement conducteur, par exemple du cuivre ou de l'aluminium ;
- le volume de la cavité est inférieur à 20 $\mu$L.

Un deuxième objet de l'invention est un procédé d'estimation d'une concentration d'un analyte dans un milieu, comportant les étapes suivantes :

a) application d'un dispositif selon le premier objet de l'invention contre un milieu, de telle sorte que la face de contact soit maintenue contre le milieu ;
b) activation de la source de lumière, la source de lumière émettant une onde lumineuse incidente impulsionnelle ou modulée en amplitude, selon une fréquence d'activation ou de modulation, dans une longueur d'onde correspondant à une longueur d'onde d'absorption de l'analyte;
c) détection d'une onde acoustique émise par le milieu, sous l'effet de l'onde lumineuse incidente, selon une fréquence acoustique égale à la fréquence d'activation de la source de lumière ;
d) mesure d'une amplitude de l'onde acoustique ;
e) détection de l'analyte et/ou estimation de la concentration de l'analyte du milieu à partir de l'amplitude mesurée lors de l'étape d).

L'invention sera mieux comprise à la lecture de l'exposé des exemples de réalisation présentés, dans la suite de la description, en lien avec les figures listées ci-dessous.

## FIGURES

Les figures 1A et 1B représentent deux modes de réalisation d'un dispositif selon l'invention.
La figure 2A représente une cavité ayant été modélisée.
La figure 2B schématise un circuit électrique équivalent de la cavité modélisée sur la figure 2A.
La figure 3A montre les résultats d'une modélisation d'une variation relative d'une amplitude d'une onde acoustique dans une cavité telle que décrite dans la figure 2A, dans la bande spectrale 100 Hz-1 kHz, en fonction de la longueur du tube débouchant de la cavité (axe des abscisses - mm), et du diamètre de ce dernier (axe des ordonnées - $\mu$m).
La figure 3B montre les résultats d'une modélisation d'une amplitude moyenne normalisée d'une onde acoustique dans une cavité telle que décrite dans la figure 2A, dans la bande spectrale 100 Hz - 1 KHz , en fonction de la longueur du tube débouchant de la cavité (axe des abscisses - mm), et du diamètre de ce dernier (axe des ordonnées - $\mu$m).
La figures 3C montrent une évolution d'une amplitude d'une onde acoustique dans la cavité (axe des ordonnées), telle que décrite dans la figure 2A, en fonction de la fréquence (axe des abscisses), en considérant respectivement différentes configurations de tubes débouchant de la cavité schématisée sur la figure 2A et modélisée par le circuit électrique schématisé sur la figure 2B.

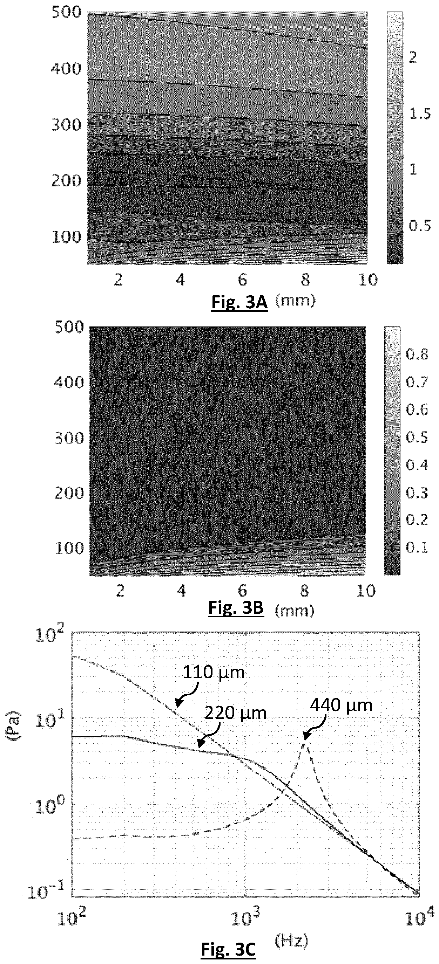

Sur les figures 3A, 3B et 3C, le volume de la cavité est égal à 5 $\mu$L.

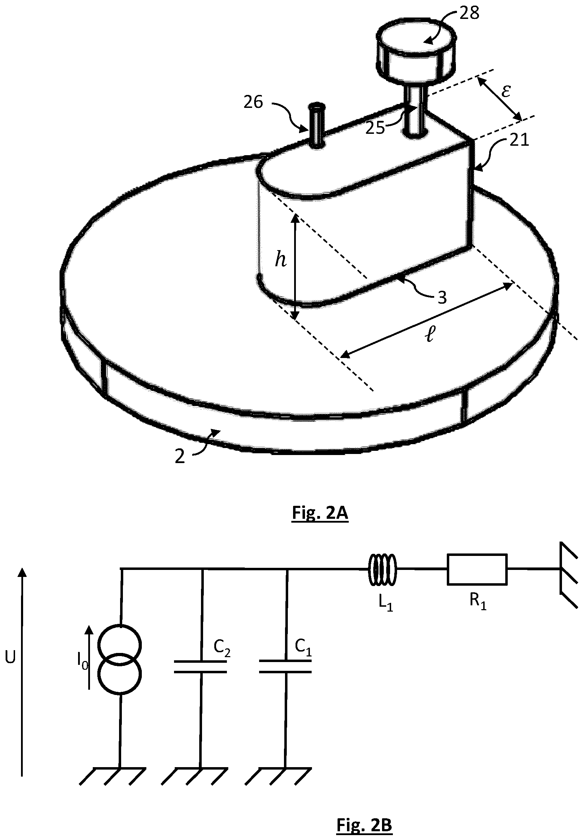

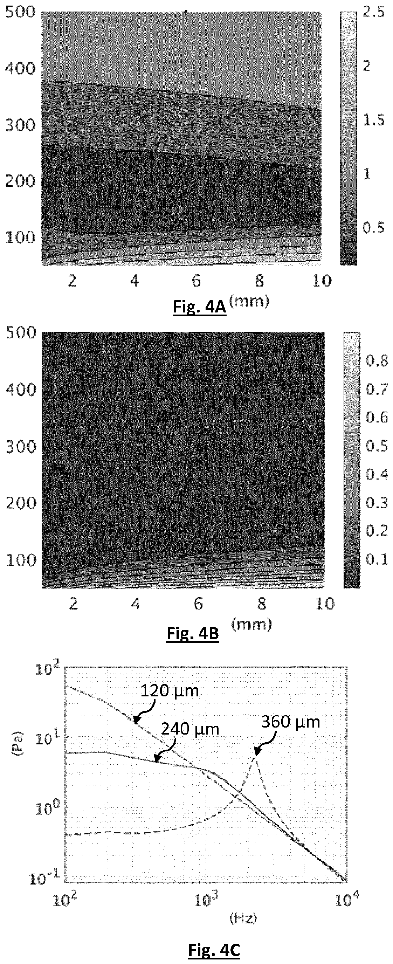

La figure 4A montre les résultats d'une modélisation d'une variation relative d'une amplitude d'une onde acoustique dans une cavité telle que décrite dans la figure 2A, dans la bande spectrale 100 Hz-1 kHz, en fonction de la longueur du tube débouchant de la cavité (axe des abscisses - mm), et du diamètre de ce dernier (axe des ordonnées - $\mu$m).
La figure 4B montre les résultats d'une modélisation d'une amplitude moyenne normalisée d'une onde acoustique dans une cavité telle que décrite dans la figure 2A, dans la bande spectrale 100 Hz - 1 KHz , en fonction de la longueur du tube débouchant de la cavité (axe des abscisses - mm), et du diamètre de ce dernier (axe des ordonnées - $\mu$m).
La figures 4C montrent une évolution d'une amplitude d'une onde acoustique dans la cavité, telle que décrite dans la figure 2A (axe des ordonnées), en fonction de la fréquence (axe des abscisses), en considérant respectivement différentes configurations de tubes débouchant de la cavité schématisée sur la figure 2A et modélisée par le circuit électrique schématisé sur la figure 2B.
Sur les figures 4A, 4B et 4C, le volume de la cavité est égal à 10 $\mu$L.

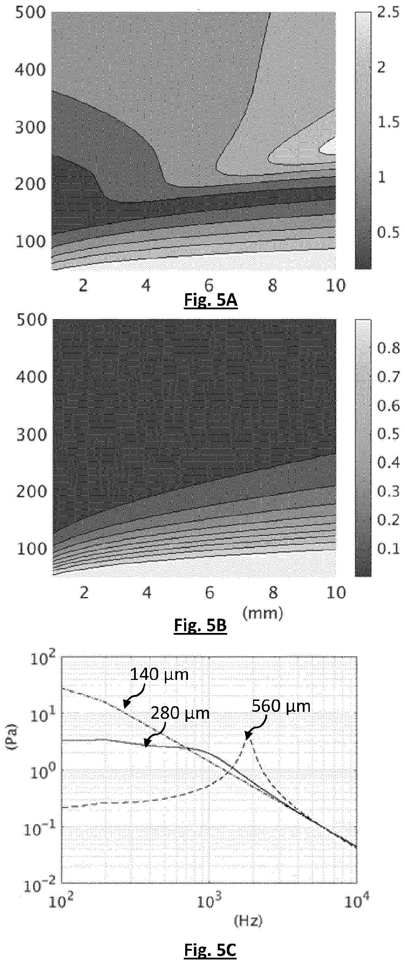

La figure 5A montre les résultats d'une modélisation d'une variation relative d'une amplitude d'une onde acoustique dans une cavité telle que décrite dans la figure 2A, dans la bande spectrale 100 Hz-1 kHz, en fonction de la longueur du tube débouchant de la cavité (axe des abscisses - mm), et du diamètre de ce dernier (axe des ordonnées - $\mu$m).
La figure 5B montre les résultats d'une modélisation d'une amplitude moyenne normalisée d'une onde acoustique dans une cavité telle que décrite dans la figure 2A, dans la bande spectrale 100 Hz - 1 KHz ,

3

en fonction de la longueur du tube débouchant de la cavité (axe des abscisses - mm), et du diamètre de ce dernier (axe des ordonnées - μm).

La figures 5C montrent une évolution d'une amplitude d'une onde acoustique dans la cavité (axe des ordonnées), telle que décrite dans la figure 2A, en fonction de la fréquence (axe des abcsisses), en considérant respectivement différentes configurations de tubes débouchant de la cavité schématisée sur la figure 2A et modélisée par le circuit électrique schématisé sur la figure 2B.

Sur les figures 5A, 5B et 5C, le volume de la cavité est égal à 20 μL.

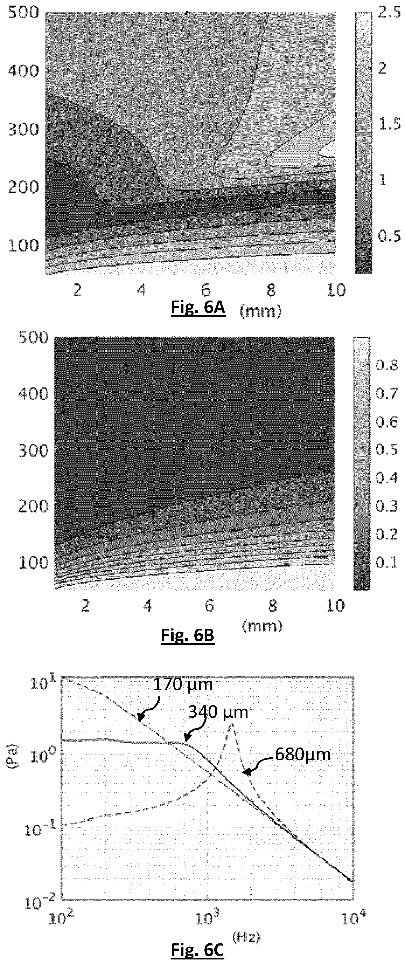

La figure 6A montre les résultats d'une modélisation d'une variation relative d'une amplitude d'une onde acoustique dans une cavité telle que décrite dans la figure 2A, dans la bande spectrale 100 Hz-1 kHz, en fonction de la longueur du tube débouchant de la cavité (axe des abscisses - mm), et du diamètre de ce dernier (axe des ordonnées - μm).

La figure 6B montre les résultats d'une modélisation d'une amplitude moyenne normalisée d'une onde acoustique dans une cavité telle que décrite dans la figure 2A, dans la bande spectrale 100 Hz - 1 KHz , en fonction de la longueur du tube débouchant de la cavité (axe des abscisses - mm), et du diamètre de ce dernier (axe des ordonnées - μm).

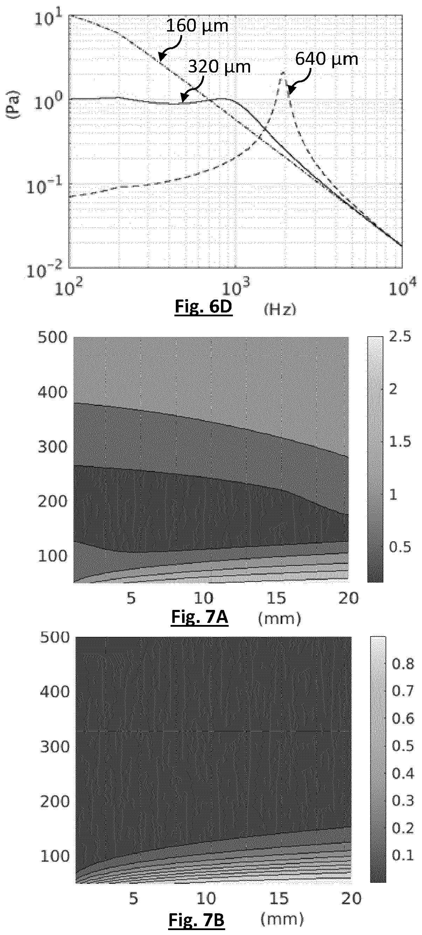

Les figures 6C et 6D montrent une évolution d'une amplitude d'une onde acoustique dans la cavité (axe des ordonnées), telle que décrite dans la figure 2A, en fonction de la fréquence (axe des abscisses), en considérant respectivement différentes configurations de tubes débouchant de la cavité schématisée sur la figure 2A et modélisée par le circuit électrique schématisé sur la figure 2B.

Sur les figures 6A, 6B, 6C, et 6D le volume de la cavité est égal à 50 μL.

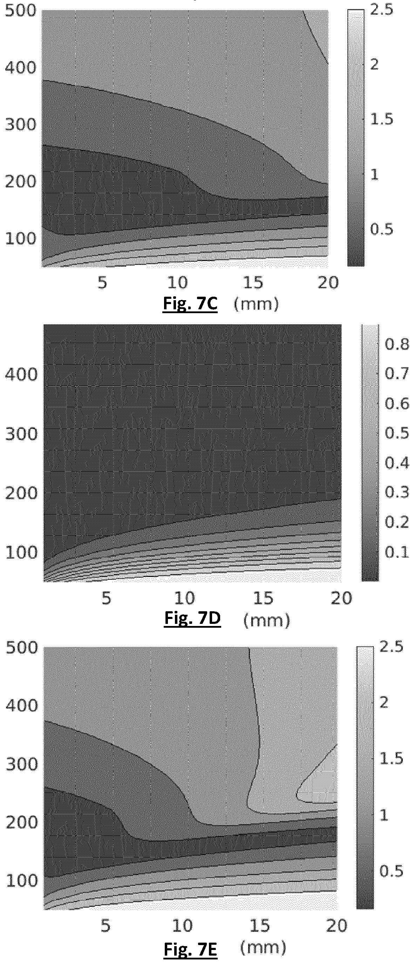

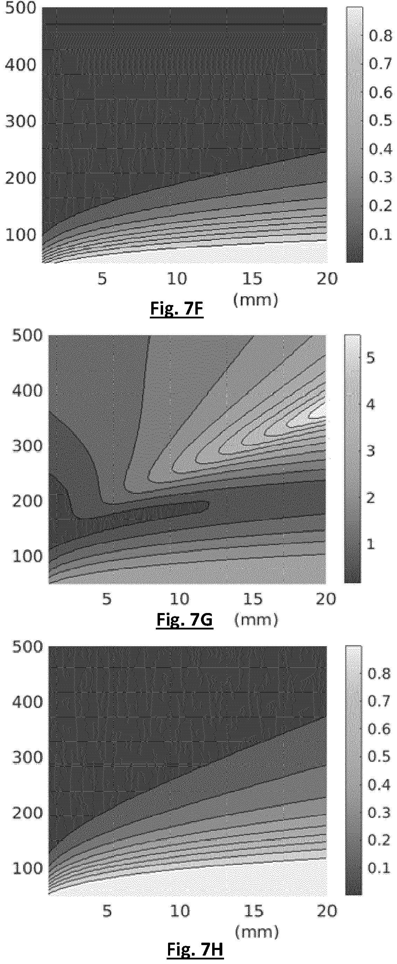

Les figures 7A, 7B, 7C, 7D, 7E,7F, 7G et 7H sont respectivement similaires aux figures 3A, 3B, 4A, 4B, 5A, 5B, 6A et 6B, en adressant une plage de longueur du tube plus étendue, variant entre 0 mm et 20 mm.

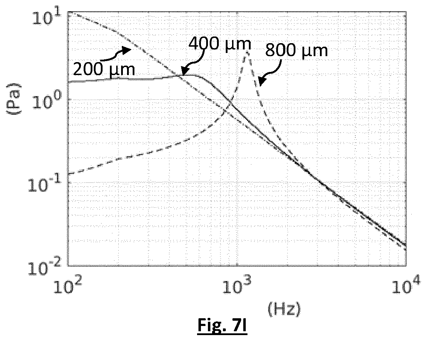

La figure 7I montre une évolution d'une amplitude d'une onde acoustique dans la cavité (axe des ordonnées), telle que décrite dans la figure 2A, de volume 50 μL, en fonction de la fréquence (axe des abscisses), en considérant respectivement différentes configurations de tubes débouchant de la cavité schématisée sur la figure 2A et modélisée par le circuit électrique schématisé sur la figure 2B. Sur la figure 7I, la hauteur du tube est de 19 mm et le volume de la cavité est de 50 μL.

## EXPOSE DE MODES DE REALISATION PARTICULIERS

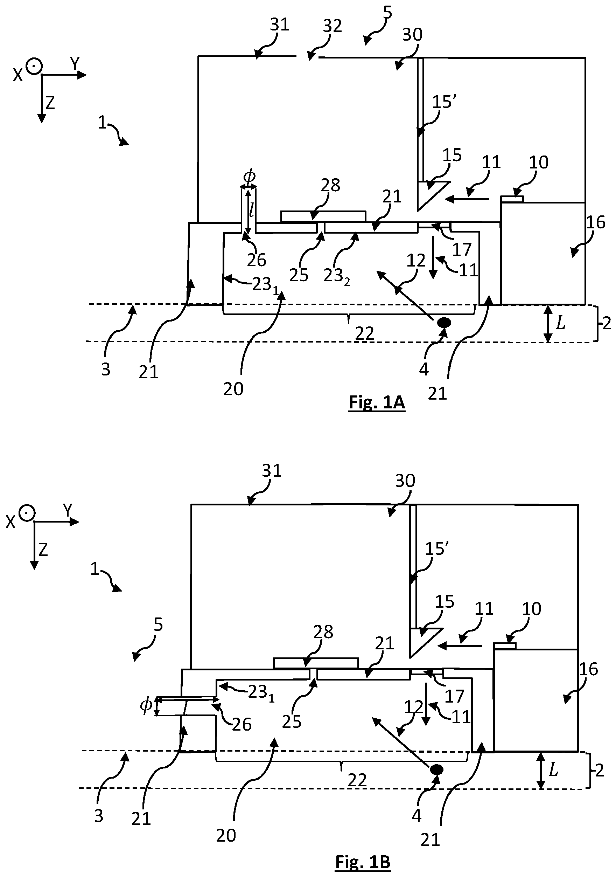

On a schématisé, sur la figure 1A, un dispositif 1 permettant une mise en oeuvre de l'invention. Le dispositif 1 est configuré pour être appliqué contre un milieu 2 à analyser. Le dispositif comporte une face de contact 3, destinée à être appliquée contre le milieu à analyser. La face de contact est conçue pour se conformer au milieu contre laquelle elle est destinée à s'appuyer. Elle est par exemple plane. Dans cet exemple, le milieu 2 est la peau d'un utilisateur. Le dispositif comporte une source de lumière 10, configurée pour émettre une onde lumineuse 11 se propageant jusqu'au milieu 2 à analyser. La source de lumière 10 est impulsionnelle ou modulée en amplitude. L'onde lumineuse 11 est émise dans une bande spectrale d'émission $\Delta\lambda$ comportant une longueur d'onde d'absorption $\lambda_a$ d'un analyte 4 présent dans le milieu. Un objectif du dispositif 1 est de détecter la présence de l'analyte 4 et éventuellement d'en estimer une concentration.

L'analyte 4 peut être une molécule présente dans un fluide corporel. Il peut par exemple s'agir de glucose, ou d'un analyte corporel de type cholesterol, triglycérides, urée, albumine, alcool (par exemple éthanol), tétrahydrocannabinol.

La bande spectrale d'émission s'étend de préférence dans le visible ou dans l'infra-rouge, par exemple entre des longueurs d'onde de 3 μm et de 15 μm. De préférence, la bande spectrale d'émission $\Delta\lambda$ est suffisamment étroite, de façon que le dispositif 1 soit spécifique à un seul analyte. Par exemple, la largeur de la bande spectrale d'émission est de l'ordre de 0.1 μm. Lorsque l'analyte est le glucose, la bande spectrale d'émission est centrée sur une longueur d'onde d'absorption du glucose, par exemple correspondant à un nombre d'onde de 1034 cm$^{-1}$. La source de lumière 10 peut notamment être une source laser impulsionnelle, par exemple un laser accordable en longueur d'onde de type QCL (Quantum Cascade Laser). La bande spectrale d'émission $\Delta\lambda$ est alors située dans l'infra-rouge.

Selon d'autres modes de réalisation, la source de lumière peut être une source de type filament, ou une diode électroluminescente. Selon ces modes de réalisation, il est préférable d'associer la source de lumière à un filtre passe-bande pour définir une bande spectrale d'émission suffisamment étroite, et centrée sur la longueur d'onde d'absorbtion considérée. Cependant, le recours à une source laser est préféré.

Dans le mode de réalisation représentés sur la figure 1A, Le dispositif 1 comporte un composant optique 15, configuré pour dévier l'onde lumineuse 11 émise par la source de lumière, vers le milieu 2 à analyser.

Le dispositif 1 est destiné à être appliqué contre le milieu à analyser 2. Il comporte une enveloppe de confinement 21, disposée au contact du milieu, et délimitant une cavité 20. La cavité 20 débouche sur une première ouverture 22, ménagée dans la face de contact 3, de façon à déboucher sur le milieu 2. L'onde lumineuse incidente 11, après avoir été réfléchie par le composant optique 15, se propage jusqu'au milieu 2 à travers la cavité 20, ainsi qu'à travers la première ouverture 22. Le dispositif comporte une fenêtre transparente 17, configurée pour transmettre l'onde lumineuse incidente 11.

Dans le dispositif représenté sur la figure 1A, le composant optique 15 est un réflecteur, prenant la forme d'un prisme réfléchissant. Il est relié à un capot 31 du dispositif par un support 15'. Dans d'autres modes de réalisation, le composant optique 15 peut être une fibre optique, décrivant une courbure, de façon que l'onde lumineuse incidente 11 parvienne jusqu'au milieu 2 selon une incidence normale, ou sensiblement normale. Par sensiblement normal, on entend normal en considérant une tolérance angulaire de $\pm$ 30 °.

Sous l'effet de la présence d'un analyte 4 dans le milieu 2, une onde photoacoustique 12 est formée. L'onde photoacoustique 12 est une onde acoustique formée à partir d'un échauffement périodique du milieu par l'onde lumineuse incidente 11, cette dernière étant impulsionnelle ou modulée en amplitude. Une partie de l'onde photoacoustique 12 s'étend à travers la cavité 20 de façon à être détectée par un transducteur acoustique 28. Le transducteur acoustique 28 est relié à la cavité 20 par un canal acoustique 25. Le transducteur acoustique peut être un microphone, présentant une gamme spectrale de détection comprenant la fréquence de l'onde photoacoustique.

Comme mentionné dans la publication Kottmann "Mid-infrared photoacoustic détection of glucose in huma skin : towards non-invasive diagnostics", Sensors 2016, 16, 1663, cette publication étant par la suite désignée "Kottmann 2016", une relation peut être établie, entre l'amplitude $A$ de l'onde photoacoustique, à la fréquence $f$ et le volume $V$ de la cavité 20, de telle sorte que :

$$A \propto \frac{I_{10}(\lambda)\alpha(\lambda)}{Vf^{\frac{3}{2}}} \quad (1)$$

où :

- $\propto$ est l'opérateur proportionnel ;
- $I_{10}(\lambda)$ est l'intensité de l'onde lumineuse incidente à la longueur d'onde $\lambda$ ;
- $\alpha(\lambda)$ est un coefficient d'absorption du milieu analysé à la longueur d'onde $\lambda$ ;
- $V$ est le volume de la cavité, incluant éventuellement le canal acoustique;
- $f$ est la fréquence de l'onde acoustique.

Lorsque la fréquence $f$ et l'intensité de l'onde lumineuse $I_{10}(\lambda)$ sont fixées, l'amplitude de l'onde photoacoustique $A$, détectée par le transducteur acoustique est proportionnelle au coefficient d'absorption du milieu $\alpha(\lambda)$. Or, ce dernier est considéré comme proportionnel à la concentration d'analyte dans le milieu. Ainsi, la mesure de l'amplitude $A$ par le transducteur acoustique 28 permet une estimation de la concentration d'analyte dans le milieu, via l'estimation du coefficient d'absorption du milieu $\alpha(\lambda)$.

L'expression (1) suppose que l'épaisseur $L$ du milieu 2 soit telle que :

$$L \gg \mu_a(\lambda) > \mu_s(\lambda) \quad (2)$$

où :

- $\mu_a(\lambda)$ est la profondeur de pénétration optique $\mu_a(\lambda)$, telle que

$$\mu_a(\lambda) = \frac{1}{\alpha(\lambda)} \quad (3)$$

- $\mu_s(\lambda)$ est une longueur de diffusion thermique, telle que

$$\mu_s(\lambda) = \left(\frac{D}{\pi f}\right)^{\frac{1}{2}} \quad (4)$$

Dans l'expression (4), D est un coefficient de diffusion thermique, tel que :

$$D = \frac{k}{\rho\, C} \quad (5)$$

où:

- $\rho$ est densité du milieu ;
- $C$ est la capacité calorifique du milieu ;
- $k$ est la conductivité thermique du milieu.

On observe que $\mu_a(\lambda)$ ne dépend pas de la fréquence $f$, tandis que $\mu_s(\lambda)$ varie selon l'inverse de la racine carrée de la fréquence $f$. Dans "Kottman 2016", il a été établi que lorsque le milieu est un tissu biologique, par exemple la peau, la condition $L \gg \mu_a(\lambda) > \mu_s(\lambda)$ est atteinte dès que la fréquence est supérieure à 50 Hz.

L'expression (1) montre que le recours à des basses fréquences, permet d'augmenter l'amplitude de l'onde photoacoustique 12. Aussi, les inventeurs ont considéré que lorsque le milieu 2 est un tissu biologique, il est avantageux que la fréquence de l'onde photoacoustique soit inférieure à 1 kHz, et de préférence inférieure à 500 Hz. Elle est de préférence comprise entre 50 Hz et 500 Hz.

D'après l'expression (4), il ressort que réduire la fréquence $f$ permet également d'augmenter la longueur de diffusion thermique $\mu_s(\lambda)$. Cela permet d'interroger le milieu davantage en profondeur.

Lorsque l'analyte 4 est le glucose, ou un autre nutriment véhiculé par le sang, l'augmentation de la profondeur, selon laquelle on détermine la concentration de l'analyte, constitue un avantage. En effet, la détection photoacoustique ne permet pas d'accéder à des profondeurs importantes, par exemple millimétriques. C'est à ces profondeurs que s'étendent les capillaires sanguins. L'estimation de la concentration du glucose par détection photoacoustique est établie indirectement, en estimant la concentration de glucose dans le fluide interstitiel, s'étendant entre les capillaires sanguins et les cellules de l'épiderme. Il a été établi que la concentration de glucose dans le fluide interstitiel correspond à la concentration de glucose dans le sang, à un décalage temporel près, de l'ordre de 20 minutes. En augmentant la profondeur selon laquelle le milieu 2 est examiné, on réduit le décalage temporel entre la concentration du glucose dans le sang et la concentration estimée en mettant en oeuvre l'expression (1).

D'après l'expression (1), le volume de la cavité 20 doit être faible pour augmenter l'amplitude de l'onde photoacoustique. Or, plus le volume de la cavité est faible, plus la fréquence $f$ est élevée.

Compte tenu de l'expression (1), un compromis est à obtenir de façon à réduire le volume tout en contrôlant la fréquence $f$, l'objectif étant d'augmenter l'amplitude de l'onde photoacoustique pour améliorer la sensibilité de la méthode.

Les inventeurs sont parvenus à un compromis satisfaisant en concevant une cavité 20 de faible volume, compris entre 1 et 50 µL, et de préférence entre 5 et 30 µL. L'enveloppe de confinement 21 comprend une paroi interne pleine 23 délimitant la cavité 20. La paroi interne comporte :

- une composante latérale $23_1$, s'étendant de préférence parallèlement à un axe normal Z à la face de contact 3.
- une composante transversale $23_2$, s'étendant parallèlement, ou sensiblement parallèlement à la face de contact 3, en regard de cette dernière. La composante transversale $23_2$ s'étend parallèlement, ou sensiblement parallèlement à la première ouverture 22. Dans les modes de réalisation représentés sur la figure 1A et 1B, la composante transversale $23_2$ inclut la fenêtre 17.

Par sensiblement parallèlement, on entend parallèle en admettant une tolérance angulaire de ± 30° ou ± 20°. La composante latérale $23_1$ s'étend entre la face de contact 3 et la composante transversale $23_2$.

Le dispositif comporte un tube 26, s'étendant à partir de la cavité 20 et débouchant sur de l'air à l'extérieur de la cavité 20. Le tube 26 forme une ouverture à travers laquelle la cavité 20 est en contact avec l'air, à la pression ambiante, à l'extérieur de la cavité. Le tube 26 s'étend selon une longueur $l$ et une section transversale de diamètre $\Phi$. Sur la figure 1A, le tube 26 s'étend à travers la composante transversale $23_2$ de la paroi interne. Sur la figure 1B, le tube 26 s'étend à travers la composante latérale $23_1$ de la paroi interne.

Sur la configuration représentée sur la figure 1A, le tube 26 débouche dans un espace 30, délimité par un capot 31. Le capot 31 comporte au moins un orifice 32 permettant une communication avec l'air 5 à l'extérieur du dispositif 1. Dans la configuration représentée sur la figure 1B, le tube 26 débouche directement sur l'air à l'extérieur du dispositif 1. Ainsi, le tube 26 débouche directement ou indirectement, sur l'air extérieur 5.

Un élément important de l'invention est le constat, par les inventeurs, qu'en faisant varier la géométrie du tube 26, la réponse spectrale de la cavité 20 varie. Les inventeurs se sont donnés l'objectif d'obtenir une réponse spectrale la plus homogène possible entre 100 Hz et 1000 Hz. Par réponse spectrale, on entend l'amplitude de la pression acoustique dans la cavité, en fonction de la fréquence.

Les inventeurs ont pris en compte une cavité telle que représentée sur la figure 2A. Les dimensions $\ell$, $\varepsilon$ et $h$ représentées sur la figure 2A étaient de 2.6 mm, 1.2 mm et 1.5 mm respectivement.

Il est connu qu'une telle cavité peut être modélisée par un modèle électrique équivalent. Cela permet de simuler la pression, à l'intérieur de la cavité, sous l'effet de la présence d'une onde acoustique, comme décrit dans la publication Dehé A. et al "The Infineon Silicon MEMS microphone", AMA conférences 2013 - Sensor 2013, Opto 2013.

On a représenté, sur la figure 2B, un modèle électrique équivalent à la cavité représentée sur la figure 2A. L'excitation par une onde acoustique d'excitation est modélisée par une source de courant $I_0$ modulée en amplitude, à une fréquence d'excitation. La réponse de la cavité, c'est-à-dire l'amplitude de l'onde acoustique dans la cavité, correspond à la tension U, également modulée en amplitude, à la fréquence d'excitation.

Si $P_0$ est la pression de l'onde acoustique dans la cavité, l'intensité $I_0$ dépend de la puissance du laser selon l'expression :

$$I_0 = \frac{\gamma - 1}{\gamma} \frac{P_{laser}}{P_0} \; \mu_s \alpha \; (6)$$

Où $\gamma$ est un coefficient adiabatique de l'air et $P_{laser}$ est la puissance du laser.

$\mu_s$ est la longueur de diffusion thermique, décrite en lien avec l'expression (4) ;

$\alpha$ est un coefficient d'absorption par unité de longueur.

L'effet de la cavité est modélisé par une capacité $C_2$, telle que :

$$C_2 = \frac{V}{\gamma P_0} \; (7)$$

où $V$ est le volume de la cavité 20, comportant éventuellement le canal acoustique 25.

L'effet du tube 26 est modélisé par une capacité $C_1$ montée en parallèle de la capacité $C_2$, ainsi qu'une inductance $L_1$ montée en série avec une résistance $R_1$, telles que :

$$C_1 = \frac{lS}{\gamma P_0} \quad (8)$$

où $l$ et $S$ correspondent respectivement à la longueur et à la section transversale du tube 26.

$$L_1 = \frac{\rho_0 l}{S} \quad (9)$$

où $\rho_0$ correspond à la masse volumique de l'air.

$$R_1 = \frac{8\pi\mu l}{S^2} \quad (10)$$

où $\mu$ correspond à la viscosité de l'air.

Les inventeurs ont utilisé le modèle schématisé sur la figure 2B, en considérant une fréquence de modulation du laser comprise entre $10^2$ Hz et $10^4$ Hz, et une puissance laser $P_{laser}$ égale à 1mW. Ils ont estimé l'amplitude de la tension U, modulée à la fréquence de modulation du laser. Elle est considérée comme représentative de la pression dans la cavité. Le calcul de la tension U, en prenant en compte différentes fréquences d'excitation, permet d'établir une réponse spectrale de la cavité 20.

Les inventeurs ont considéré successivement 4 volumes différents de cavité, respectivement égaux à 5 µL, 10 µL, 20 µL et 50 µL. Pour chaque volume, ils ont étudié l'effet de la variation du diamètre du tube et de sa longueur, sur la réponse spectrale de la cavité. Les performances de chaque configuration modélisée ont été examinées en considérant :

- une variation relative de la pression $\Delta P_0$ dans la bande spectrale 100 Hz - 1 kHz : Elle correspond à l'expression suivante :

$$\Delta P_0 = \frac{P_{0,max} - P_{0,min}}{\overline{P_0}} \quad (11)$$

où $P_{0,max}$, $P_{0,min}$ et $\overline{P_0}$ sont respectivement la pression maximale la pression minimale et la pression moyenne dans la bande spectrale 100 Hz - 1 kHz. L'objectif est d'obtenir une variation relative de la pression la plus faible possible.

- une pression moyenne normalisée par une pression moyenne obtenue en mettant en oeuvre une cavité fermée, c'est-à-dire sans tube 26. La pression moyenne normalisée est notée $P_{0,N}$. Elle est telle que :

$$\overline{P_{0,N}} = \frac{\overline{P_0}}{\overline{P_{0,C}}} \quad (12)$$

où $\overline{P_0}$ et $\overline{P_{0,C}}$ sont respectivement la pression moyenne dans la cavité modélisée, dans la bande spectrale 100 Hz - 1 KHz, et la pression moyenne, dans une cavité de même dimension, mais fermée, dans la même bande spectrale. La pression moyenne normalisée représente l'intensité du signal détecté, et correspond à un indicateur relatif à la sensibilité de la mesure. Il est préférable qu'elle soit la plus élevée possible.

Pour déterminer $\Delta P_0$ et $\overline{P_{0,N}}$, les inventeurs ont modélisé une cavité telle que décrite en lien avec la figure 2A. Ils ont considéré différents volumes et, pour chaque volume, différentes dimensions du tube : en particulier un diamètre variant entre 50 µm et 500 µm et une longueur variant entre 1 mm et 10 mm. Ils ont modélisé la cavité selon le modèle électrique décrit en lien avec la figure 2B. Ils ont estimé la tension U, représentative de la pression $P_0$, pour différentes fréquences, comprises entre 100 Hz et 10 kHz. A partir de ces modélisations, ils ont estimé les grandeurs $\Delta P_0$ et $\overline{P_{0,N}}$, décrites en lien avec les expressions (11) et (12), ces grandeurs étant considérées comme des indicateurs de performance.

Les figures 3A et 3B représentent respectivement la variation relative de la pression $\Delta P_0$ et la pression moyenne normalisée $\overline{P_{0,N}}$ en fonction de la longueur du tube 26 (axe des abscisses - unité mm) et le diamètre du tube (axe des ordonnées - unité µm). Le volume de la cavité est de 5 µL. Sur chaque figure, la valeur de la grandeur considérée correspond au niveau de gris. La figure 3C représente la réponse spectrale modélisée en considérant un volume de la cavité égal à 5 µl, la longueur du tube étant de 10 mm, pour différents diamètres de tube, respectivement égaux à 110 µm, 220 µm, 440 µm. Il résulte que les performances optimales (faible valeur $\Delta P_0$ et valeur $\overline{P_{0,N}}$ élevée) sont obtenues lorsque la longueur du tube est supérieure à 3 mm ou 5 mm et que le diamètre du tube est compris entre 150 µm et 300 µm.

L'analyse de la figure 3C montre que lorsque le diamètre du tube est faible (110 µm), la réponse spectrale est proche de celle d'une cavité fermée. Lorsque le diamètre s'élève à 220 µm, la réponse spectrale peut être considérée comme relativement "plate", c'est-à-dire uniforme dans une bande spectrale comprise entre 100 Hz et 1000 Hz, ce qui correspond à l'objectif recherché. La valeur de la pression $P_0$ est suffisamment élevée pour être aisément mesurable. Lorsque le diamètre augmente

encore (360 $\mu$m), la réponse spectrale de la cavité se rapproche d'une réponse spectrale d'une cavité résonante, avec une apparition d'un pic de résonance. La valeur de la pression $P_0$ diminue, ce qui la rend plus difficilement mesurable.

Les figures 4A, 4B et 4C sont respectivement similaires aux figures 3A, 3B et 3C, en prenant en compte un volume de cavité égal à 10 $\mu$L. Sur la figure 4C, les diamètres des tubes considérés sont de 120 $\mu$m, 240 $\mu$m et 360 $\mu$m, la longueur étant de 9.4 mm. On en déduit que les performances optimales (faible valeur $\Delta P_0$ et valeur $\overline{P_{0,N}}$ élevée) sont obtenues lorsque la longueur du tube est supérieure à 3 mm ou 5 mm et que le diamètre du tube est compris entre 150 $\mu$m et 300 $\mu$m.

Les figures 5A, 5B et 5C sont respectivement similaires aux figures 3A, 3B et 3C, en prenant en compte un volume de cavité égal à 20 $\mu$L. Sur la figure 5C, les diamètres des tubes considérés sont de 140 $\mu$m, 280 $\mu$m et 560 $\mu$m, la longueur étant de 9.6 mm. On en déduit que les performances optimales (faible valeur $\Delta P_0$ et valeur $\overline{P_{0,N}}$ élevée) sont obtenues lorsque la longueur du tube est supérieure à 2 mm ou 5 mm et que le diamètre du tube est compris entre 200 $\mu$m et 350 $\mu$m.

Les figures 6A, 6B et 6C sont respectivement similaires aux figures 3A, 3B et 3C, en prenant en compte un volume de cavité égal à 50 $\mu$L. Sur la figure 6C, les diamètres des tubes considérés sont de 170 $\mu$m, 340 $\mu$m et 680 $\mu$m, la longueur étant de 9.4 mm. La figure 6D représente la réponse spectrale modélisée en considérant un volume de la cavité égal à 50 $\mu$L, la longueur du tube étant de 5 mm, pour différents diamètres de tube, respectivement égaux à 160 $\mu$m, 320 $\mu$m et 640 $\mu$m.

On déduit des figures 6A à 6D, que les performances optimales (faible valeur $\Delta P_0$ et valeur $\overline{P_{0,N}}$ élevée) sont obtenues lorsque la longueur du tube est supérieure à 1 mm ou 5 mm et que le diamètre du tube est compris entre 250 $\mu$m et 500 $\mu$m.

Les figures 7A, 7B, 7C, 7D, 7E,7F, 7G et 7H sont respectivement similaires aux figures 3A, 3B, 4A, 4B, 5A, 5B, 6A et 6B, en adressant une plage de longueur du tube plus étendue, variant entre 0 mm et 20 mm. Ces figures montrent que les plages de valeurs de diamètre de tube précédemment définies s'appliquent jusqu'au moins une longueur de tube *l* atteignant 20 mm. La figure 7I représente la réponse spectrale modélisée en considérant un volume de la cavité égal à 50 $\mu$L, la longueur du tube étant de 19 mm, pour différents diamètres de tube, respectivement égaux à 200 $\mu$m, 400 $\mu$m et 800 $\mu$m. De même qu'en lien avec les figures 6C et 6D, on observe que les performances optimales (faible valeur $\Delta P_0$ et valeur $\overline{P_{0,N}}$ élevée) sont obtenues, lorsque la longueur du tube atteint 19 mm, pour un diamètre du tube compris entre 250 $\mu$m et 500 $\mu$m.

D'une façon générale, quel que soit le volume considéré, lorsque le diamètre du tube est faible la réponse spectrale est proche de celle d'une cavité fermée. Lorsque le diamètre du tube est élevé, la réponse spectrale de la cavité se rapproche d'une réponse spectrale d'une cavité résonante, avec une apparition d'un pic de résonance. Les plages de diamètres et de longueur mentionnées ci-dessus correspondent à l'obtention d'une réponse spectrale relativement plate, entre la réponse spectrale représentative d'une cavité fermée et une réponse spectrale représentative d'une cavité résonante.

En se basant sur les modélisations effectuées, les inventeurs ont déterminé, pour différentes plages de volumes de cavité, les dimensions optimales du tube :

- Lorsque le volume de la cavité est inférieur ou égal à 15 $\mu$L, le diamètre du tube peut être compris entre 150 $\mu$m et 300 $\mu$m.
- Lorsque le volume de la cavité est compris entre 15 $\mu$L et 30 $\mu$L, le diamètre du tube peut être compris entre 200 $\mu$m et 350 $\mu$m.
- Lorsque le volume de la cavité est supérieur à 30 $\mu$L, le diamètre du tube peut être compris entre 250 $\mu$m et 500 $\mu$m.

Quel que soit le volume la longueur du tube est de préférence supérieure à 1 mm ou à 3 mm. Elle est de préférence inférieure à 20 mm.

Dans les modes de réalisation représentés sur les figures 1A et 1B, la source de lumière 10 est une source laser de type QCL, formée sur un substrat. Pour des raisons de simplicité de conception, il est préférable que l'onde lumineuse émise par la source de lumière s'étende parallèlement au plan du substrat dans laquelle elle est formée. Le dispositif comporte un composant otique 15 pour diriger tout ou partie de l'onde lumineuse incidente 11 vers la première ouverture 22. Le composant optique peut être un réflecteur ou une fibre optique.

La température optimale de fonctionnement des sources laser de type QCL est généralement comprise entre 30°C et 40°C. Lorsque le dispositif est appliqué contre la peau d'un utilisateur vivant, la température corporelle de l'utilisateur peut être utilisée en tant que source de chaleur de la source laser QCL. Le dispositif comporte alors un support 16, contre lequel la source laser 10 est disposée. Le support 16 est configuré pour entrer en contact avec la peau de l'utilisateur. Le support est réalisé selon un matériau présentant de bonnes propriétés de conduction thermique, par exemple un métal, et notamment du cuivre ou de l'aluminium. Le support 16 s'étend entre la face de contact 3 et la source de lumière 10. L'épaisseur du support 16, selon l'axe Z, est par exemple comprise entre 1 mm et 10 mm. Le support 16 agit ainsi en tant que tampon thermique, entre la peau de l'utilisateur et la source de lumière.

L'invention pourra être mise en oeuvre pour détecter la présence d'un analyte dans un milieu, ce dernier pouvant être la peau d'un utilisateur. Pour cela, les étapes suivantes seront appliquées :

- application du dispositif contre un milieu, de telle sorte que la face de contact soit maintenue contre le milieu ;

- activation de la source de lumière selon une fréquence d'activation ou une fréquence de modulation;
- détection d'une onde photoacoustique émise par le milieu, sous l'effet de l'onde lumineuse incidente, selon une fréquence acoustique égale à la fréquence d'activation ou de modulation de la source de lumière ;
- mesure d'une amplitude de l'onde acoustique, ce qui revient à estimer l'amplitude $A$ décrite en lien avec l'expression (1) ;
- détection de la présence de l'analyte et/ou estimation de la concentration en analyte du milieu à partir de l'amplitude mesurée. Cette étape est effectuée en estimant l'absorption $\alpha$ décrite en lien avec l'expression (1), la relation entre la concentration d'analyte et l'absorption étant connue.

**Revendications**

1. Dispositif de détection photoacoustique (1), destiné à être appliqué, par une face de contact (3), contre un milieu à analyser, le dispositif comportant :

   - une cavité creuse (20), débouchant sur une première ouverture (22), la première ouverture étant ménagée dans la face de contact;
   - une source de lumière (10), impulsionnelle ou modulée en amplitude, configurée pour émettre une onde lumineuse incidente (11), dans une bande spectrale d'émission ($\Delta\lambda$), à travers la cavité (20), jusqu'à la première ouverture ;
   - un transducteur acoustique (28) relié à la cavité, et configuré pour détecter une onde photoacoustique (12) s'étendant à travers la cavité ;
   de telle sorte que sous l'effet d'une illumination du milieu, par l'onde lumineuse incidente, le transducteur acoustique détecte une onde acoustique produite par un échauffement du milieu (2);
   le dispositif étant **caractérisé en ce que** :

   - le volume de la cavité (20) est inférieur à 50 $\mu$L ;
   - le dispositif comporte un tube ouvert (26), s'étendant à partir de la cavité (20) et débouchant sur de l'air s'étendant à l'extérieur de la cavité, selon une longueur comprise entre 1 et 20 mm, le diamètre du tube étant tel que :

     • lorsque le volume de la cavité est inférieur ou égal à 15 $\mu$L, le diamètre du tube est compris entre 150 $\mu$m et 300 $\mu$m ;
     • lorsque le volume de la cavité est compris entre 15 $\mu$L et 30 $\mu$L, le diamètre

du tube est compris entre 200 $\mu$m et 350 $\mu$m ;
     • lorsque le volume de la cavité est supérieur à 30 $\mu$L, le diamètre du tube est compris entre 250 $\mu$m et 500 $\mu$m.

2. Dispositif selon la revendication 1, dans lequel :

   - la cavité est délimitée par une paroi transversale ($23_1$) et une paroi latérale ($23_2$), la paroi latérale s'étendant entre la paroi transversale et la face de contact ;
   - le tube s'étend à travers la paroi transversale ou à travers la paroi latérale.

3. Dispositif selon l'une quelconque des revendications précédentes, comportant un canal acoustique (25), s'étendant entre le transducteur acoustique (28) et la cavité (20).

4. Dispositif selon l'une quelconque des revendications précédentes, comportant un composant optique (15) configuré pour diriger l'onde lumineuse incidente (11), émise par la source de lumière (10), vers la première ouverture (22).

5. Dispositif selon la revendication 4, dans lequel le composant optique est un réflecteur.

6. Dispositif selon l'une quelconque des revendications 4 ou 5, dans lequel la source de lumière est une source laser.

7. Dispositif selon la revendication 6, le dispositif comportant un support (16), s'étendant de la face de contact (3) jusqu'à la source de lumière (10), le support étant configuré pour conduire une chaleur émise par le milieu analysé, vers la source de lumière.

8. Dispositif selon la revendication 7, dans lequel le support comporte un métal thermiquement conducteur, par exemple du cuivre ou de l'aluminium.

9. Dispositif selon l'une quelconque des revendications précédentes, dans lequel le volume de la cavité est inférieur à 20 $\mu$L.

10. Procédé d'estimation d'une concentration en analyte d'un milieu, comportant les étapes suivantes :

    a) application d'un dispositif (1) selon l'une quelconque des revendications précédentes contre un milieu (2), de telle sorte que la face de contact (3) soit maintenue contre le milieu ;
    b) activation de la source de lumière (10), la source de lumière émettant une onde lumineuse incidente impulsionnelle ou modulée en amplitude, selon une fréquence d'activation ou de modulation, dans une longueur d'onde correspondant à une longueur d'onde d'absorption de l'analyte ;

c) détection d'une onde acoustique (12) émise par le milieu, sous l'effet de l'onde lumineuse incidente, selon une fréquence acoustique égale à la fréquence d'activation de la source de lumière ;

d) mesure d'une amplitude de l'onde acoustique ;

e) détection de la présence d'analyte et/ou estimation de la concentration de l'analyte du milieu à partir de l'amplitude mesurée lors de l'étape d).


**Patentansprüche**

1. Photoakustische Detektionsvorrichtung (1) zur Applikation auf ein zu analysierendes Medium über eine Kontaktfläche (3), wobei die Vorrichtung Folgendes aufweist:

   - einen hohlen Hohlraum (20), der an einer ersten Öffnung (22) mündet, wobei die erste Öffnung in der Kontaktfläche vorgesehen ist;

   - eine gepulste oder amplitudenmodulierte Lichtquelle (10), die dazu ausgebildet ist, eine einfallende Lichtwelle (11) in einem Emissionsspektralband ($\Delta\lambda$) durch den Hohlraum (20) bis zur ersten Öffnung zu emittieren;

   - einen Schallwandler (28), der mit dem Hohlraum verbunden ist und dazu ausgebildet ist, eine photoakustische Welle (12) zu detektieren, die sich durch den Hohlraum erstreckt;

   so dass unter der Einwirkung einer Illumination des Mediums durch die einfallende Lichtwelle der Schallwandler eine Schallwelle detektiert, die durch eine Erwärmung des Mediums (2) erzeugt wird;

   wobei die Vorrichtung **dadurch gekennzeichnet ist, dass**:

      - das Volumen des Hohlraums (20) kleiner als 50 $\mu$L ist;

      - die Vorrichtung eine offene Röhre (26) aufweist, die sich ausgehend von dem Hohlraum (20) erstreckt und in Luft mündet, die sich außerhalb des Hohlraums erstreckt, über eine Länge zwischen 1 und 20 mm, wobei der Durchmesser der Röhre so ist, dass:

         • wenn das Volumen des Hohlraums kleiner als oder gleich 15 $\mu$L ist, der Durchmesser der Röhre zwischen 150 $\mu$m und 300 $\mu$m beträgt;

         • wenn das Volumen des Hohlraums zwischen 15 $\mu$L und 30 $\mu$L beträgt, der Durchmesser der Röhre zwischen 200 $\mu$m und 350 $\mu$m beträgt;

         • wenn das Volumen des Hohlraums größer als 30 $\mu$L ist, der Durchmesser der Röhre zwischen 250 $\mu$m und 500 $\mu$m beträgt.

2. Vorrichtung nach Anspruch 1, wobei:

   - der Hohlraum von einer Querwand ($23_1$) und einer Seitenwand ($23_2$) begrenzt ist, wobei sich die Seitenwand zwischen der Querwand und der Kontaktfläche erstreckt;

   - sich die Röhre durch die Querwand oder durch die Seitenwand erstreckt.

3. Vorrichtung nach einem der vorhergehenden Ansprüche, die einen akustischen Kanal (25) aufweist, der sich zwischen dem Schallwandler (28) und dem Hohlraum (20) erstreckt.

4. Vorrichtung nach einem der vorhergehenden Ansprüche, die eine optische Komponente (15) aufweist, die dazu ausgebildet ist, die einfallende Lichtwelle (11), die von der Lichtquelle (10) emittiert wird, zur ersten Öffnung (22) hin zu lenken.

5. Vorrichtung nach Anspruch 4, wobei die optische Komponente ein Reflektor ist.

6. Vorrichtung nach einem der Ansprüche 4 oder 5, wobei die Lichtquelle eine Laserquelle ist.

7. Vorrichtung nach Anspruch 6, wobei die Vorrichtung einen Träger (16) aufweist, der sich von der Kontaktfläche (3) bis zur Lichtquelle (10) erstreckt, wobei der Träger dazu ausgebildet ist, eine Wärme, die vom analysierten Medium abgegeben wird, zur Lichtquelle zu leiten.

8. Vorrichtung nach Anspruch 7, wobei der Träger ein wärmeleitendes Metall aufweist, zum Beispiel Kupfer oder Aluminium.

9. Vorrichtung nach einem der vorhergehenden Ansprüche, wobei das Volumen des Hohlraums kleiner als 20 $\mu$L ist.

10. Verfahren zur Schätzung einer Analytkonzentration eines Mediums, das die folgenden Schritte aufweist:

   a) Applizieren einer Vorrichtung (1) nach einem der vorhergehenden Ansprüche auf ein Medium (2), so dass die Kontaktfläche (3) an das Medium gehalten wird;

   b) Aktivieren der Lichtquelle (10), wobei die Lichtquelle eine gepulste oder amplitudenmodulierte einfallende Lichtwelle emittiert, mit einer Aktivierungs- oder Modulationsfrequenz, in einer Wellenlänge, die einer Absorptionswellenlänge des Analyten entspricht;
c) Detektieren einer Schallwelle (12), die von dem Medium unter der Einwirkung der einfallenden Lichtwelle mit einer akustischen Frequenz abgegeben wird, die gleich der Aktivierungsfrequenz der Lichtquelle ist;
d) Messen einer Amplitude der Schallwelle;
e) Detektieren des Vorhandenseins von Analyt und/oder Schätzen der Konzentration des Analyten des Mediums anhand der beim Schritt d) gemessenen Amplitude.

**Claims**

1. Photoacoustic detecting device (1), intended to be applied, via a contact face (3), against a medium to be analysed, the device comprising:

   - a hollow cavity (20) that emerges onto a first aperture (22), the first aperture being produced in the contact face;
   - a pulsed or amplitude-modulated light source (10) configured to emit, in an emission spectral band ($\Delta\lambda$), an incident light wave (11) through the cavity (20) to the first aperture;
   - an acoustic transducer (28) linked to the cavity and configured to detect a photoacoustic wave (12) extending through the cavity;
   such that, under the effect of illumination of the medium, by the incident light wave, the acoustic transducer detects an acoustic wave produced by heating of the medium (2);
   the device being **characterized in that**:

   - the volume of the cavity (20) is smaller than 50 $\mu$L;
   - the device comprises an open tube (26) extending between the cavity (20) and the air lying outside the cavity, over a length comprised between 1 and 20 mm, the diameter of the tube being such that:

      • when the volume of the cavity is smaller than or equal to 15 $\mu$L, the diameter of the tube is comprised between 150 $\mu$m and 300 $\mu$m;
      • when the volume of the cavity is comprised between 15 $\mu$L and 30 $\mu$L, the diameter of the tube is comprised between 200 $\mu$m and 350 $\mu$m;
      • when the volume of the cavity is larger than 30 $\mu$L, the diameter of the tube is comprised between 250 $\mu$m and 500 $\mu$m.

2. Device according to Claim 1, wherein:

   - the cavity is bounded by a transverse wall ($23_1$) and a lateral wall ($23_2$), the lateral wall extending between the transverse wall and the contact face;
   - the tube extends through the transverse wall or through the lateral wall.

3. Device according to either one of the preceding claims, comprising an acoustic channel (25) extending between the acoustic transducer (28) and the cavity (20).

4. Device according to any one of the preceding claims, comprising an optical component (15) configured to direct the incident light wave (11), emitted by the light source (10), to the first aperture (22).

5. Device according to Claim 4, wherein the optical component is a reflector.

6. Device according to either one of Claims 4 and 5, wherein the light source is a laser source.

7. Device according to Claim 6, the device comprising a holder (16) extending from the contact face (3) to the light source (10), the holder being configured to conduct heat emitted by the analysed medium to the light source.

8. Device according to Claim 7, wherein the holder comprises a thermally conductive metal, copper or aluminium for example.

9. Device according to any one of the preceding claims, wherein the volume of the cavity is smaller than 20 $\mu$L.

10. Method for estimating a concentration of analyte in a medium, comprising the following steps:

    a) applying a device (1) according to any one of the preceding claims against a medium (2), such that the contact face (3) is held against the medium;
    b) activating the light source (10), the light source emitting, at a wavelength corresponding to an absorption wavelength of the analyte, an incident light wave that is pulsed or amplitude-modulated at an activation or modulation frequency;
    c) detecting an acoustic wave (12) emitted by the medium, under the effect of the incident light wave, at an acoustic frequency equal to the activation frequency of the light source;
    d) measuring an amplitude of the acoustic wave;
    e) detecting the presence of analyte and/or estimating the concentration of the analyte in the medium from the amplitude measured in step d).

**Fig. 1A**

**Fig. 1B**

**Fig. 2A**

**Fig. 2B**

Fig. 3A (mm)

Fig. 3B (mm)

Fig. 3C (Hz)

**Fig. 4A** (mm)

**Fig. 4B** (mm)

**Fig. 4C**

**Fig. 5A**

**Fig. 5B**

**Fig. 5C**

Fig. 6A (mm)

Fig. 6B (mm)

Fig. 6C (Hz)

**Fig. 6D**  (Hz)

**Fig. 7A**  (mm)

**Fig. 7B**  (mm)

**Fig. 7C** (mm)

**Fig. 7D** (mm)

**Fig. 7E** (mm)

Fig. 7F

Fig. 7G

Fig. 7H

**Fig. 7I**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2008011055 A1
- US 2019159705 A1

**Littérature non-brevet citée dans la description**

- **BAUER AJ.** IR-spectroscopy for skin in vivo : Optimal skin sites and properties for non-invasive glucose measurement by photoacoustic and photothermal spectroscopy. *Journal of biopohtonics,* 2018, vol. 11
- Windowless ultrasound photoacoustic cell for in-vivo mid-IR spectroscopy of human epidermis : Low interférence by changes of air pressure, température, and humidity caused by skin contact opens the possibility for a non-invasive monitoring of glucose in the interstitial fluid. *Rev. Sci. Instrum.,* 2013, vol. 84, 084901
- **KOTTMANN.** Mid-infrared photoacoustic détection of glucose in huma skin : towards non-invasive diagnostics. *Sensors,* 2016, vol. 16, 1663
- **DEHÉ A. et al.** The Infineon Silicon MEMS microphone. *AMA conférences 2013 - Sensor 2013, Opto,* 2013